# EUROPEAN PATENT APPLICATION

(11) **EP 3 957 297 A1**
(43) Date of publication of application: **23.02.2022**
(21) Application number: 20192238.2
(22) Date of filing: 21.08.2020
(51) Int. Cl.: A61K 8/44, A61K 8/46, A61Q 19/10

(54) **PERSONAL CLEANSING COMPOSITION**

(71) Applicant: PZ Cussons (International) Limited, Manchester M22 5TG (GB)
(72) Inventor: Heffernan, Paul, deceased (GB); Wu, Maggie, Bromley Cross Bolton BL7 9XS (GB); DAVIES, Craig, Stoke-on-Trent, Staffordshire, ST7 2XG (GB); WU, Meiki, Bolton BL7 9XS (GB)
(74) Representative: Wilson Gunn

(57) **Abstract**

The present invention relates to a composition for use in a personal care or cleansing composition, particularly body washes and hand washes, or shampoo, foam bath, or a head-to-toe baby cleanser, which is mild on the skin or provides a minimal irritation potential to the skin as well as the eyes; has a pleasant product texture or rheology; has a pleasant skin feel during and after washing; and has good foaming properties, with a rapid lather generation, a large amount of lather generated, and a good richness of lather; and is relatively inexpensive to manufacture.

## Description

The present invention relates to a composition for use in a personal care or cleansing composition, particularly body washes and hand washes, or shampoo, foam bath, or a head-to-toe baby cleanser.

### Background to the invention

Products for personal care or personal cleansing have been known for a long time. They are usually surfactant-based compositions, and a commonly used anionic surfactant in such compositions is sodium lauryl ether sulfate (abbreviated to SLES, or also called sodium laureth sulfate under the International Nomenclature for Cosmetic Ingredients (INCI) standardised naming system).

However, there is a growing movement towards 'green' or 'natural' personal care or cleansing compositions, which employ more natural compounds. Such green compositions avoid the use of SLES, as while green certifying bodies (*e.g.* COSMOS) accept sulphation (also known as sulphatation) as a chemical process and therefore inclusion of sulfates as ingredients, they do not accept alkoxylation (which includes ethoxylation) as a process and therefore do not accept ethoxylated ingredients. Therefore, alkyl sulfate surfactants, such as sodium lauryl sulfate (SLS) or sodium coco-sulfate (SCS) would be permitted as they do not involve ethoxylation of the fatty alcohol feedstock prior to sulphation. They are also considered more 'natural' as the carbon content is entirely from renewable sources.

There are a number of such 'green' personal care or cleansing products which are commercially available. However, these products tend to have deficiencies in either mildness (as they are soap-based or contain alkyl sulfate liquids), or in their in-use performance, such as in lathering (including the ease of generating lather, as well as the amount and texture), as well as the ability of spreading neat product on wet skin and the feel on the skin (in wash and post-wash). Deficiencies in one or more of these sensory qualities gives a poor level of consumer acceptance. These deficiencies, coupled with high pricing, has resulted in a low level of consumer uptake.

Current personal care or cleansing products marketed as 'green' options can be grouped into six categories based upon their primary surfactant technologies, each with their own individual advantages and disadvantages. It should be noted that co-surfactants are nearly always employed, including other anionic surfactants, amphoteric surfactants and nonionic surfactants. Cocamidopropyl betaine (CAPB) is a popular choice of co-surfactant in all categories because it offers a number of technical benefits: it provides a creamy lather, reduces the irritation caused by anionic surfactants, and helps to build viscosity. It is also accepted by certifying bodies, is widely available from many vendors, and is relatively low cost.

The six categories of 'green' options can be described as below:

| | **Technology** | **Advantages** | **Disadvantages** |
|---|---|---|---|
| **1** | Soap based liquids (typically potassium counter-ion) | Fully renewable carbon content. Good cleansing and foaming. Can be organic certified. Approved by 'green' certifying bodies. | Can only be made to high pH (>9.0). Drying and irritant to skin. Undesirable skin feel (for regions with temperate climate). Complex to manufacture (hot process). |
| **2** | Alkyl Sulfate-based (as primary surfactant). Typically SLS, ALS or Sodium Cocosulfate (broad alkyl distribution). | Fully renewable carbon content (assuming alkyl is plant origin which it usually is). Good cleansing and foaming. Easy to thicken. Approved by 'green' certifying bodies due to absence of petrochemical inputs. | Strongly irritant to eyes and skin. Stripped skin-feel. Can't claim sulfate-free status. Very open foam, so poor lather quality and foam density perceived as lacking in richness. |
| **3** | Alkyl Ether Sulfate-based (as primary surfactant). Typically SLES or ALES. | Low cost, good cleansing and foaming, easy to thicken (with salt), standard processing, milder than alkyl sulfates (although are still moderately irritating). These are classical formulations dressed up to appear 'natural' (use of graphics, botanical tipins, fragrance selection etc.) | Not differentiated from regular product bases. Has a significant proportion of non-renewable carbon from ethylene oxide. Contamination concerns (1,4-dioxane). Not approved by certifying bodies. Can't claim sulfate-free status. |
| **4** | Olefin Sulfonate-based | Enables 'free-from' claims (sulfates, SLS, SLES). Excellent foaming. Easy to process and thicken. Low cost. | Petrochemical-derived, so cannot make 'natural' claims. More irritant than ether sulfates. Not approved by certifying bodies. |
| **5** | Isethionate based | Enables 'free-from' claims (sulfates, SLS, SLES). Largely naturally derived but part petrochemical-derived. Milder than alkyl ether sulfates. Good foaming. Easy to thicken in combination with CAPB. | Poor solubility and hydrolytic stability of isethionates. Can be improved via methylation (e.g. SLMI) but more expensive. Not |
| | | | approved by certifiers as it is a sulfonate. |
| **6** | Complex surfactant blends - covers a multitude of surfactant chemistries including betaines, amino acid surfactants, alkyl polyglucosides etc. | Generally enable 'free-from' claims as above. Mild formulations generally. | High cost, complex to manufacture, sometimes contain PEG based thickeners (1,4-dioxane issue) or natural gums (undesirable rheology). Typically have poor foaming performance. |

| | | | |
|---|---|---|---|
| ALS = ammonium lauryl sulfate ALES = ammonium lauryl ether sulfate SCI = sodium cocoyl isethionate SLMI = sodium lauroyl methyl isethionate | | | |

Therefore, it would be desirable to provide a 'green' personal care or cleansing composition which does not exhibit the disadvantages of the existing commercial products, *i.e.* a composition which does not contain sulfates or alkoxylates and is based on surfactants that are substantially plant derived (substantially meaning >50% by either carbon fraction or molecular weight contribution, as defined in ISO 16128).

Additionally, the composition is mild on the skin or provides a minimal irritation potential to the skin as well as the eyes; has a pleasant product texture or rheology (*i.e.* it feels syrupy and spreads easily); has a pleasant skin feel during and after washing; has good foaming properties, with a rapid lather generation, a large amount of lather generated, and a good richness of lather; and which is relatively low cost to manufacture.

The present invention is able to provide such a composition.

Therefore, in accordance with the invention, there is provided a composition comprising:
A) an acyl sarcosinate;
B) an alkylamidopropyl betaine; and
C) a sulfonated fatty acid.

The present invention is a base for a personal care or personal cleansing formulation, such as a shower gel or hand washing product, which offers a high level of sensory and foaming performance, is milder than the first 4 categories listed above, is free of sulfate surfactants and ethylene oxide derivatives, and is also free of 'external' thickening agents, and is easy and relatively low cost to manufacture.

Acyl sarcosinates, such as sodium lauryl sarcosinate, have been found to be particularly useful as a replacement for conventional anionic surfactants such as sodium lauryl ether sulfate, because they have excellent foaming characteristics (they provide richer lather than SLES), are known to be milder than SLES, are resistant to foam collapse from hard water or sebum, and, due to their substantivity to skin and hair, impart a light conditioning effect. They are also readily biodegradable, so do not present any environmental concerns.

As carboxylate surfactants, acyl sarcosinates have properties that may be pH dependent. At lower pH values, the carboxyl group may become protonated, so an increasing proportion may exist in the non-ionic (undissociated) form. Foaming is optimal in the mildly acidic range. The solubility is also reduced at lower pH values but this can be a useful feature in the presence of amphoteric surfactants as it is believed (while not being bound to any particular theory) that this can favour the formation of larger surfactant aggregates such as worm-like micelles which, due to their entanglement, cause an increase in viscosity of the system.

Alkylamidopropyl betaines have been found to be particularly useful in combination with acyl sarcosinates.

At acidic pH, the betaines become protonated and thus gain a net positive charge *(i.e.* take on cationic character). This results in a stronger interaction with the anionic form of the sarcosinate, contributing to the viscosity increase. As pH is lowered to a certain point, the amount of sarcosinate remaining in the anionic form is diminished so the viscosity reaches a peak value and then collapses quickly as further acid is added, typically resulting in turbidity due to the poor solubility of the free acid form of the sarcosinate. In practical terms, this is seen as a very steep viscosity *vs* pH curve, and this is a real challenge for formulators and for scale-up.

Non-limiting examples of suitable acids that may be employed to induce the pH reduction include mineral acids (hydrochloric, sulphuric, phosphoric etc) or organic (lactic, formic, ethanoic, propionic, citric, oxalic etc).

The inventors have found that the viscosity maximum occurs within the pH range of about 4.5 to about 5.5, and within this range the peak value will depend on the precise ratio of the sarcosinate and betaine.

It is possible to formulate products using the above combination of sarcosinate and betaine. However, the inventors found that it is not possible to achieve a sufficiently high viscosity (comparable to market norms) when other formulation ingredients (such as preservatives and fragrances) are incorporated, while keeping the surfactant amount at reasonable and cost effective levels *(i.e.* about 10 wt%), as the other formulation ingredients tend to lower the viscosity of this system. While it may be possible to achieve the required viscosity at much higher levels of the surfactants, this increases the costs to unrealistic levels.

The use of non-ionic surfactants to boost viscosity was investigated; while this was successful in terms of the viscosity properties, it had a negative impact on the foaming qualities.

However, the inventors surprisingly found that inclusion of a sulfonated fatty acid had the desired effect of both increasing viscosity and improving foaming attributes simultaneously.

The composition may be considered as a surfactant system comprising the components (A)-(C).

According to one embodiment of the invention, the acyl sarcosinate may be present in an amount of between about 2 and about 10 wt% of the composition of the invention, typically 3-7 wt%, more preferably 4-6 wt%.

The alkylamidopropyl betaine may also be present in an amount of between about 2 and about 10 wt% of the composition of the invention, typically 4-8 wt%, more preferably 5-7 wt%.

The sulfonated fatty acid may be present in an amount of between about 0.2 and about 5.0 wt% of the composition of the invention, typically about 0.3-2.0 wt%.

The total of the components (A)-(C) is typically about 8-20 wt% of the composition of the invention, more typically about 9-15 wt%.

The pH of the composition may be adjusted to be within the range 4.5-5.5, typically 4.7-5.3. This pH adjustment may be carried out using a suitable Bronsted acid.

Further discussion of the components of the composition of the invention is provided below.

### Constituent A - Acyl Sarcosine

Acyl sarcosines are the condensation products of a reaction between the amino acid sarcosine (N-methyl glycine) and a natural fatty acid. Commercially they are made by reacting sodium sarcosine with the parent fatty acid chloride, the lauroyl type typically being made from fatty acids derived from palm kernel oil. The sodium sarcosinate salt is generated by a simple neutralisation with sodium hydroxide. Sarcosine is a naturally occurring amino acid in both animals and plants, as an intermediate in both glycine synthesis and degradation. Commercially it is manufactured synthetically so can be considered 'nature-identical'.

In one aspect of the invention, the acyl sarcosinate may be selected from one or more compounds of the formula: 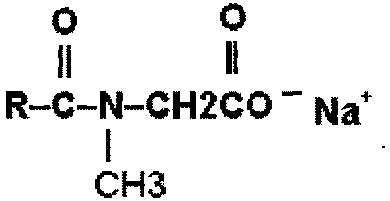 wherein R = a C₇-C₂₄ alkyl or alkenyl group.

The corresponding potassium, ammonium and triethanolammonium salts of these sodium acyl sarcosinates may also be used.

In one aspect of the invention, the acyl sarcosinate may be selected from one or more of sodium lauroyl sarcosinate, ammonium lauroyl sarcosinate, sodium cocoyl sarcosinate, sodium myristoyl sarcosinate, sodium oleyl sarcosinate, and an un-neutralised sarcosine.

The equivalent acid forms of these materials (acyl sarcosines) are also available, so the salts could be generated in situ with a suitable neutralising agent.

### Constituent B - Alkylamidopropyl Betaine

Alkylamidopropyl betaines of the general formula below are extensively used in personal cleansing products of all types because of their mildness, their ability to mitigate the irritation potential of anionic surfactants, their excellent foaming qualities, ability to build viscosity via formation of mixed micelles (especially with alkyl sulfates and alkyl ether sulfates) that respond well to electrolyte addition, and the fact they are widely available commercially. Typically, they are used as co-surfactants together with primary anionic surfactants, but it has been known to use them as primary surfactants in areas where mildness is the key consideration, such as in face washes. Additionally, they are readily biodegradable and considered an acceptable choice by 'green' certifying organisations such as COSMOS.

In one aspect of the invention, the alkylamidopropyl betaine may be selected from one or more compounds of the formula: 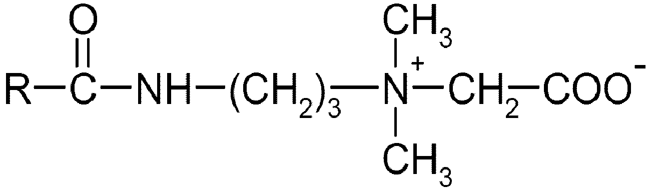 wherein R = a C₇-C₂₄ alkyl or alkenyl group.

Betaines of this type are typically manufactured via one of two routes. They can either be oil derived (made directly from triglycerides) or fatty acid derived (using refined fatty acids which are typically derived from vegetable oil sources). Both routes involve a 2-stage reaction process carried out in separate reaction vessels.

The first stage is the reaction of the oil or fatty acids with dimethylaminopropylamine (DMAPA) to form an amidoamine. When triglycerides are used as feedstock, glycerol is produced as a by-product, and this is left in. When fatty acids are used as the feedstock, water is produced as a by-product, but this needs to be distilled off as the reaction process needs to be anhydrous to go to completion.

The second stage is the same for both routes and involves reaction of the amidoamine intermediate (produced in the first stage) with either monochloroacetic acid (or its sodium salt) in an aqueous environment. Sodium chloride is produced as a by-product.

Commercially, betaines are available from either route. Typically, if from the oil route, coconut oil or palm kernel oil is used. Coconut oil is the major feedstock in oil derived CAPB. If made from the fatty acid route the major source tends to be palm kernel oil.

Alkylamidopropyl betaines are available from many of the major surfactant producers such as BASF, Innospec, Evonik, Clariant, Kao and Solvay.

The alkylamidopropyl betaine used in the present invention may be selected from one or more of cocamidopropyl betaine, lauramidopropyl betaine, myristamidopropyl betaine, oleamidopropyl betaine, and mixtures or any two or more thereof.

### Constituent C - Sulfonated Fatty Acid

The third surfactant in the present invention is a sulfonated fatty acid. Although their synthesis was first described in the 1920's, this class of surfactants has not found widespread use in personal cleansing compositions due to difficulty in making them to an acceptably light colour, and their low water solubility also presents challenges in the formulation of liquids.

They are manufactured by reacting a fatty acid with sulphur trioxide in a falling-film reactor, followed by neutralisation with sodium hydroxide, and then bleached to an acceptable colour. Examples of sulfonated fatty acids which may be useful in the present invention include those derived from lauric acid.

The sulfonated fatty acid used in the present invention may have the general formula: 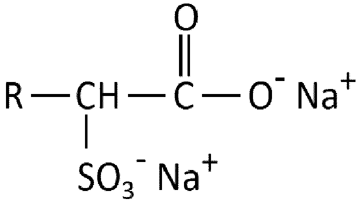 wherein R = a C₆-C₂₂ alkyl or alkenyl group.

The sulfonated fatty acid used in the present invention may be selected from one or more of disodium 2-sulfolaurate, disodium 2-sulfocaprylate, disodium 2-sulfocaprate, disodium 2-sulfomyristate, disodium 2-sulfocetylate and disodium 2-sulfooleate, and mixtures or any two or more thereof.

The sulfonated fatty acid may be disodium 2-sulfolaurate, which has the formula:

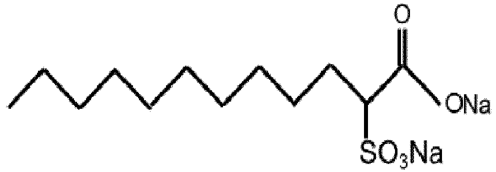

This material is available either as a single substance in solid paste form (Texapon SFA, manufactured by BASF, Germany) or pre-blended with cocamidopropyl betaine (Dehyton SFA, also from BASF) in liquid form.

It is based on 100% renewable carbon content; is readily biodegradable; improves foaming properties of primary surfactants, as it has finer bubbles which leads to a creamier lather; and is exceptionally mild to skin and eyes in combination with cocamidopropyl betaine.

In the present invention, the sulfonated fatty acid raises the viscosity of the surfactant system within the claimed pH range and gives measurably improved foam volume and texture in both in vitro and in vivo tests.

All three of the surfactants (A)-(C) are mild surfactants. Thickening is a key challenge in SLES-free surfactant systems, as one cannot just use salt.

In the present invention, no additional thickener is required. This avoids other issues with the properties of the composition, and also keeps costs to a minimum. Thickeners typically used in similar compositions are either natural gums (which lead to poor flow properties and sensory performance) or are highly ethoxylated materials (*e.g*. polyethylene glycols) which are not suitable for a 'natural' composition.

According to one embodiment of the invention, the acyl sarcosinate comprises or is sodium lauroyl sarcosinate; the alkylamidopropyl betaine comprises or is cocamidopropyl betaine; and the sulfonated fatty acid comprises or is disodium 2-sulfolaurate.

The composition of the invention may further contain one or more ingredients selected from emollients, moisturizers, conditioners, skin conditioners, hair conditioners, vitamins, pro-vitamins, antioxidants, free-radical scavengers, dyes, hair coloring agents, bleaching agents, UV absorbers, anti-UV agents, antibacterial agents, antifungal agents, melanin regulators, tanning accelerators, depigmenting agents, skin lightening agents, skin-coloring agents, liporegulators, weight-reduction agents, anti-acne agents, antiseborrhoeic agents, anti-ageing agents, anti-wrinkle agents, keratolytic agents, anti-inflammatory agents, anti-acne agents, antibiotics, anti-inflammatory agents, botanical extracts, imidazoles, refreshing agents, cicatrizing agents, vascular-protection agents, agents for the reduction of dandruff, seborrheic dermatitis, or psoriasis, shale oil and derivatives thereof, anti-psoriasis agents, corticosteroids depilating agents, agents for combating hair loss, reducing agents for permanent-waving, reflectants, essential oils and fragrances.

The various components of the composition of the invention are added in their respective amounts as desired; the balance of the composition to 100% is water. The water content is typically at least about 80 wt%.

The viscosity of the composition is ideally at least about 5000 cP, when measured on a Brookfield viscometer (spindle 3 at 5 rpm) at 20°C.

The following laboratory-based test method was used to assess the foaming ability and resulting lather richness of the compositions.
1. A Kenwood BL740 food blender was used to generate lather as follows: the blender jug was placed on an electronic 2-decimal place balance and warm tap water adjusted to 40±2°C (160.0 g) was weighed directly into this.
2. The test formulation (40.0 g) was then added directly to the water in the blender jug.
3. The 4:1 mixture was then blended on speed 4 for 30 seconds and then stopped (timed using a stop clock, which was left running).
4. The lather created was immediately poured into a 2000 ml measuring cylinder (using a silicone spatula to scrape as much as possible from the blender jug) and the foam volume determined visually using the graduations.
5. The lather in the measuring cylinder from the lather volume test was left to stand until the stop clock reached 5 minutes and 30 seconds, *i.e.* exactly 5 minutes since the blender was switched off.
6. At this time point the amount of liquor that had drained (visible at the base of cylinder) was determined using the graduations (x).

The amount of liquor draining from the lather is indicative of lather instability and inversely related to the perceived richness of lather (unpublished internal study). By this measure, a totally unstable lather (that drained/collapsed fully within 5 minutes) would result in the full amount of liquor used to create the lather (*i.e.* 200 ml) being visible at the base of the cylinder; whereas a totally stable lather would result in no visible liquor. An index between these two extremes thus provides a useful measure of lather richness. This can be done using the simple calculation: Lather Richness Index (LRI) = (200-x)/2.

It is desirable for the LRI to have a high value, *i.e.* greater than 50 and preferably greater than 60.

The composition of the present invention functions effectively and exhibits the desired properties outlined above without needing to use specific ingredient types - such as sulphate-based surfactants, alkoxylated substances (as surfactants, thickeners or solubilisers), soaps *(i.e.* simple fatty acid salts). No surfactants in which the carbon content is entirely of non-renewable origin (*i.e*. petrochemical-derived, such as olefin sulfonates, alkyl benzene sulfonates, or synthetic ether sulfates) are required.

Also provided in accordance with the present invention is a method of manufacturing a composition comprising:
A) an acyl sarcosinate;
B) an alkylamidopropyl betaine; and
C) a sulfonated fatty acid;
the method comprising adding an amount of a sulfonated fatty acid to an acyl sarcosinate or an alkylamidopropyl betaine; or adding an amount of a sulfonated fatty acid to an aqueous mixture of an acyl sarcosinate and an alkylamidopropyl betaine.

The method may also include adjusting the pH of the mixture containing all of the components (A)-(C) to an appropriate level, such as between 4.5 and 5.5 using an appropriate acid. One or more further ingredients selected from the list above may also be added.

Also provided in accordance with the present invention is a use of a composition comprising:
A) an acyl sarcosinate;
B) an alkylamidopropyl betaine; and
C) a sulfonated fatty acid
as a personal cleansing composition, such as a body wash, facial wash or hand wash, or for washing hair.

The present invention will be further described with reference to the following examples, which are intended to be illustrative only, and in no way limiting upon the scope of the invention.

### EXAMPLES

### Examples A to C:

Examples A to C were prepared as simple surfactant blends (10% by weight total surfactant content), figures are as wt% active matter, and the pH was adjusted to circa 5.0 in each case. A reference blend (classic 70/30 SLES/CAPB) is included for comparative purposes, this does not fall within the present invention. Each ingredient was added to deionised water at ambient temperature in the order listed, with stirring, and mixed until fully dissolved. The lather evaluation testing was performed in duplicate and the average results reported in the table below.

| Ingredient | Supplier | Reference | A | B | C |
|---|---|---|---|---|---|
| Sodium Laureth Sulfate | BASF | 7.00 | | | |
| Sodium Lauroyl Sarcosinate | Croda | | 4.75 | 4.50 | 4.00 |
| Cocamidopropyl Betaine | Solvay | 3.00 | 4.75 | 4.50 | 4.00 |
| Disodium 2-sulfolaurate # | BASF | | 0.50 | 1.00 | 2.00 |
| Sodium Chloride | | 2.40 | | | |
| Citric acid monohydrate | Brenntag | | 0.70 | 0.70 | 0.75 |
| Sodium Hydroxide 10% w/w | | 0.02 | | | |
| Water (deionised) | | Balance | Balance | Balance | Balance |
| pH at 20°C | | 4.97 | 4.97 | 5.00 | 4.98 |
| Viscosity* | | 8,620 | 10,300 | 9,580 | 21,400 |
| Foam vol (ml) | | 1,050 | 1,335 | 1,390 | 1,420 |
| Lather Richness Index | | 40.0 | 55.0 | 61.3 | 72.5 |

| | | | | | |
|---|---|---|---|---|---|
| #Supplied in semi-solid paste form (tradename Texapon SFA). Warmed to 45°C before use to facilitate handling. *Brookfield spindle 3, 5rpm, 20°C | | | | | |

The results show that the formulations A to C, conforming to the present invention, demonstrate much better foaming performance, in terms of both volume and richness, than the conventional reference formulation that has the same active matter content.

### Example D:

This is an example of a fully formulated composition conforming to the present invention. Of the materials in the form they were supplied, the amounts used in the composition of the invention were as shown in Table 1 below:

**Table 1**

| **Ingredient** | **% w/w Inclusion** | **Supplier** |
|---|---|---|
| Sodium Lauroyl Sarcosinate 30% w/w | 16.67 | Croda |
| Cocamidopropyl Betaine 30% w/w | 16.67 | Solvay |
| Cocamidopropyl Betaine (and) Disodium 2-Sulfolaurate 40%* | 3.00 | BASF |
| Sodium Benzoate solution 30% | 1.33 | Univar |
| Fragrance compound - OS Pineapple & Jalapeno 1771501 | 0.26 | Seven Scent |
| Citric acid monohydrate 50% solution | 1.40 | Brenntag |
| Water | Balance | |

| | | |
|---|---|---|
| *Tradename Dehyton SFA, consists of CAPB and disodium 2-sulfolaurate (both materials are palm kernel oil derived) in a 3:2 ratio at approximately 40% total active matter content. | | |

Of the materials in terms of the active ingredients therein, the amounts used were as shown in Table 2 below:

**Table 2**

| **Ingredient** | **% w/w Active Matter** | **Supplier** |
|---|---|---|
| Sodium Lauroyl Sarcosinate | 5.00 | Croda |
| Cocamidopropyl Betaine | 5.72 | Solvay |
| Disodium 2-Sulfolaurate 40%* | 0.48 | BASF |
| Sodium Benzoate (granular) | 0.40 | Univar |
| Fragrance compound - OS Pineapple & Jalapeno 1771501 | 0.26 | Seven Scent |
| Citric acid monohydrate (solid) | 0.70 | Brenntag |
| Water | Balance | |

### Method of Manufacture

The above formulation example was made as follows:
1. The formulation quantity of public utility supply tap water was added to a glass beaker at ambient temperature.
2. Cocamidopropyl betaine was added to the water with gentle stirring to avoid foaming.
3. Sodium lauroyl sarcosinate was added to the above mixture with gentle stirring.
4. The blend of disodium 2-sulfolaurate and cocamidopropyl betaine was added to the above and stirred gently until fully dissolved.
5. Sodium benzoate solution was added and stirred gently until dispersed.
6. Fragrance was added and stirred gently until fully dissolved. The liquid was clear and very low viscosity at this stage.
7. Citric acid solution was added slowly while continuously monitoring the pH of the formulation using an electronic pH meter.
8. The liquid was observed to thicken as pH was lowered to below 5.5, at this point the stirrer speed was gradually increased to ensure good mixing. After the full amount of citric acid had been added the resulting viscous liquid was stirred for a further 15 minutes.

Final product pH was 5.09.
Viscosity (Brookfield spindle 3, speed set to 5 rpm) = 8,850 cP
Lather volume = 1450 ml
Lather Richness Index = 70

It is of course to be understood that the present invention is not intended to be restricted to the foregoing example, which is described by way of illustration only.

## Claims

1. A composition comprising:
A) an acyl sarcosinate;
B) an alkylamidopropyl betaine; and
C) a sulfonated fatty acid.

2. A composition according to claim 1, wherein the acyl sarcosinate is present in an amount of between about 2 and about 10 wt% of the composition.

3. A composition according to claim 1 or claim 2, wherein the alkylamidopropyl betaine is present in an amount of between about 2 and about 10 wt% of the composition.

4. A composition according to any preceding claim, wherein the sulfonated fatty acid is present in an amount of between about 0.2 and about 5.0 wt% of the composition.

5. A composition according to any preceding claim, wherein the total of the components (A)-(C) is about 8-20 wt% of the composition.

6. A composition according to any preceding claim, wherein the acyl sarcosinate comprises one or more compounds of the formula: 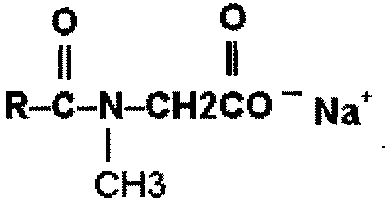 wherein R = a C₇-C₂₄ alkyl or alkenyl group;
or compounds of the same formula with the corresponding ammonium salt.

7. A composition according to claim 6, wherein the acyl sarcosinate may be selected from one or more of sodium lauroyl sarcosinate, ammonium lauroyl sarcosinate, sodium cocoyl sarcosinate, sodium myristoyl sarcosinate, or sodium oleyl sarcosinate; or the corresponding potassium, ammonium or triethanolammonium salts.

8. A composition according to any preceding claim, wherein the alkylamidopropyl betaine comprises one or more compounds of the formula: 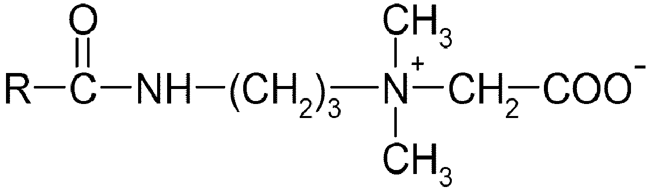 wherein R = a C₇-C₂₄ alkyl or alkenyl group.

9. A composition according to claim 8, wherein the alkylamidopropyl betaine is selected from one or more of cocamidopropyl betaine, lauramidopropyl betaine, myristamidopropyl betaine, oleamidopropyl betaine, and mixtures or any two or more thereof.

10. A composition according to any preceding claim, wherein the sulfonated fatty acid comprises one or more compounds of the formula: 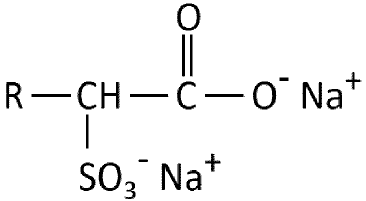 wherein R = a C₆-C₂₂ alkyl or alkenyl group.

11. A composition according to any preceding claim, wherein the sulfonated fatty acid is selected from one or more of disodium 2-sulfolaurate, disodium 2-sulfocaprylate, disodium 2-sulfocaprate, disodium 2-sulfomyristate, disodium 2-sulfocetylate and disodium 2-sulfooleate, and mixtures or any two or more thereof.

12. A composition according to any preceding claim, wherein the acyl sarcosinate comprises or is sodium lauroyl sarcosinate; the alkylamidopropyl betaine comprises or is cocamidopropyl betaine; and the sulfonated fatty acid comprises or is disodium 2-sulfolaurate.

13. A composition according to any preceding claim, wherein the pH of the composition is between about 4.5 to about 5.5; and/or
wherein no additional thickener is required; and/or
further comprising one or more ingredients selected from emollients, moisturizers, conditioners, skin conditioners, hair conditioners, vitamins, pro-vitamins, antioxidants, free-radical scavengers, abrasives, dyes, hair coloring agents, bleaching agents, UV absorbers, anti-UV agents, antibacterial agents, antifungal agents, melanin regulators, tanning accelerators, depigmenting agents, skin lightening agents, skin-coloring agents, liporegulators, weight-reduction agents, anti-acne agents, antiseborrhoeic agents, anti-ageing agents, anti-wrinkle agents, keratolytic agents, anti-inflammatory agents, anti-acne agents, antibiotics, anti-inflammatory agents, botanical extracts, imidazoles, refreshing agents, cicatrizing agents, vascular-protection agents, agents for the reduction of dandruff, seborrheic dermatitis, or psoriasis, shale oil and derivatives thereof, anti-psoriasis agents, corticosteroids depilating agents, agents for combating hair loss, reducing agents for permanent-waving, reflectants, essential oils and fragrances.

14. A method of manufacturing a composition comprising:
A) an acyl sarcosinate;
B) an alkylamidopropyl betaine; and
C) a sulfonated fatty acid;
the method comprising adding an amount of a sulfonated fatty acid to an acyl sarcosinate or an alkylamidopropyl betaine; or adding an amount of a sulfonated fatty acid to an aqueous mixture of an acyl sarcosinate and an alkylamidopropyl betaine;
and optionally further comprising adjusting the pH of the mixture containing all of the components (A)-(C) to between 4.5 and 5.5 using an acid.

15. Use of a composition according to any of claims 1-13 as a personal care or cleansing composition.
